# EUROPEAN PATENT SPECIFICATION

(11) **EP 4 170 668 B1**
(45) Date of publication and mention of the grant of the patent: **18.10.2023**
(21) Application number: 21306474.4
(22) Date of filing: 22.10.2021
(51) Int. Cl.: G16H 20/40

(54) **DEVICE AND METHOD FOR NEAR REAL-TIME PREDICTION OF AN IRRADIATION MAP FOR INTERVENTIONAL RADIOLOGY**
VORRICHTUNG UND VERFAHREN ZUR NAH-ECHTZEITVORHERSAGE EINER BESTRAHLUNGSKARTE FÜR INTERVENTIONELLE RADIOLOGIE
DISPOSITIF ET PROCÉDÉ DE PRÉDICTION EN TEMPS QUASI RÉEL D'UNE CARTE D'IRRADIATION POUR LA RADIOLOGIE INTERVENTIONNELLE

(43) Date of publication of application: 26.04.2023
(73) Proprietor: Institut National de la Santé et de la Recherche Médicale, 75013 Paris (FR); Universite Brest Bretagne Occidentale, 29200 Brest (FR); Centre Hospitalier Régional et Universitaire de Brest, 29200 Brest (FR); Université de Strasbourg, 67000 Strasbourg (FR); Centre National de la Recherche Scientifique, 75016 Paris (FR)
(72) Inventor: Bert, Julien, 29200 Brest (FR); Villa Arias, Matéo, 29200 Brest (FR); Padoy, Nicolas, 67000 Strasbourg (FR); Visvikis, Dimitris, 29200 Brest (FR)
(74) Representative: Novagraaf Technologies

(56) References cited:
- BOUZID DOUNIA ET AL: "Monte-Carlo dosimetry for intraoperative radiotherapy using a low energy x-ray source", ACTA ONCOLOGICA., vol. 54, no. 10, 24 March 2015 (2015-03-24), pages 1788-1795, XP55908246, GB ISSN: 0284-186X, DOI: 10.3109/0284186X.2015.1016623
- TANABE HIROYOSHI ET AL: "Fast EUV lithography simulation using convolutional neural network", JOURNAL OF MICRO/NANOLITHOGRAPHY, MEMS, AND MOEMS, SPIE - INTERNATIONAL SOCIETY FOR OPTICAL ENGINEERING, US, vol. 20, no. 4, 1 October 2021 (2021-10-01), page 41202, XP060149677, ISSN: 1932-5150, DOI: 10.1117/1.JMM.20.4.041202 [retrieved on 2021-09-24]
- AURALEE EDELEN ET AL: "Machine Learning for Orders of Magnitude Speedup in Multi-Objective Optimization of Particle Accelerator Systems", ARXIV.ORG, CORNELL UNIVERSITY LIBRARY, 201 OLIN LIBRARY CORNELL UNIVERSITY ITHACA, NY 14853, 19 March 2019 (2019-03-19), XP081581226,
- PENG ZHAO ET AL: "A method of rapid quantification of patient-specific organ doses for CT using deep-learning-based multi-organ segmentation and GPU-accelerated Monte Carlo dose computing", MEDICAL PHYSICS., vol. 47, no. 6, 3 April 2020 (2020-04-03), pages 2526-2536, XP55911075, US ISSN: 0094-2405, DOI: 10.1002/mp.14131 Retrieved from the Internet: URL:https://onlinelibrary.wiley.com/doi/fu ll-xml/10.1002/mp.14131>
- SHAN HONGMING ET AL: "Synergizing medical imaging and radiotherapy with deep learning", MACHINE LEARNING: SCIENCE AND TECHNOLOGY, vol. 1, no. 2, 23 June 2020 (2020-06-23), page 021001, XP055881802, DOI: 10.1088/2632-2153/ab869f Retrieved from the Internet: URL:https://iopscience.iop.org/article/10. 1088/2632-2153/ab869f>

## Description

### Technical Field

Various example embodiments relate to interventional radiology.

It applies in particular to the prevention of risks linked to irradiation due to this type of radiology, by allowing a quasi-real time prediction of an irradiation map of the intervention environment.

### Background

Interventional radiology refers to all medical procedures performed by radiologists under radiological control, allowing the treatment or invasive diagnosis of numerous pathologies. The principle of interventional radiology is therefore to access a lesion located inside the body in order to carry out a diagnostic (sampling, for example) or therapeutic act (aimed at treating, repairing, closing, etc.).

Thus, in the context of therapeutic intervention, interventional radiology represents in certain cases an alternative to conventional surgical treatment, by making it possible to intervene inside the body, via the natural channels and without making surgical openings

Different technologies can be used such as, for example, fluoroscopy, X-ray scanning, ultrasound scanning, MRI (magnetic resonance imaging), etc.

Interventional radiology therefore involves irradiating portions of the human body from within the body and for the duration of the procedure. It therefore poses a radiation risk to patients. Thus, prolonged exposure to X-rays can cause deterministic side effects (skin bonding, hair loss, cataracts, etc.) and non-deterministic side effects (cancers in particular).

The International Commission on Radiological Protection (ICRP) and other bodies have emphasised the importance of patient dose control during interventional radiology.

Imaging systems have been commercialised with functions for real-time measurement of metrics such as Dose Area Product (DAP). Examples of such systems are Toshiba's Dose Tracking System, GE Healthcare's DoseMap and Siemens' CAREwatch.

However, these systems do not take into account the specific anatomy of the patient, or even the position and orientation of the interventional radiology device. The information provided is therefore necessarily approximate. Furthermore, they estimate the dose only on the skin surface.

Another family of proposals is based on a more complex simulation of the dose, in particular according to a Monte-Carlo method. The simulation can take into account different input parameters characterising the patient and the X-ray device, but it suffers from a very high computation time that makes it unsuitable for real time. It is therefore not possible to use such an approach to allow a practitioner to control the dose imposed on the patient during a procedure.

It has been proposed to deploy Monte Carlo simulation methods on dedicated computing processors such as Graphics Processing Units (GPUs).

An example of published paper is Badal A, Badano A. « Accelerating Monte Carlo simulations of photon transport in a voxelized geometry using a massively parallel graphics processing unit » in Med Phys. 2009 Nov;36(11):4878-80. doi: 10.1118/1.3231824. PMID: 19994495, or, J. Bert, H. Perez-Ponce, Z. El Bitar, S. Jan, Y. Boursier, et al.. « Geant4-based Monte Carlo simulations on GPU for medical applications » in Physics in Medicine and Biology, IOP Publishing, 2013, 58, pp.5593-5611.

These proposals do indeed reduce calculation times, but they remain inappropriate for real time, since they are of the order of 10 seconds for the first proposal (which, moreover, does not take into account the specific anatomy of the patient) and 2 minutes for the second proposal.

Further proposals are beginning to appear that take advantage of recent advances in the field of multilayer neural networks. These include Roser, P., Zhong, X., Birkhold, A., Strobel, N., Kowarschik, M., Fahrig, R., & Maier, A. (2019). "Physics-Driven Learning of X-ray Skin Dose Distribution in Interventional Procedures" in Medical physics, 46, 4654-4665.

However, this approach is also not workable for several reasons: it relies on ray tracing pre-processing which also makes it expensive in terms of computation time and not suitable for real-time. The article indicates a time of about 2 seconds for the calculation of an irradiation map. Moreover, the accuracy of the estimation is not very good since it gives an average error of 8% with peaks of 22% and furthermore, the map focuses on the most irradiated areas, thus reducing the information provided to the practitioner. Also, the method relies on a three-dimensional model of each exposed organ of the patient to proceed with the learning of the predictive model. In practice, this type of information is not widely available, which can only hinder the building of a learning set to build an effective predictive model. In fact, in the article, the learning is performed only on 3 "phantoms".

BOUZID DOUNIA ET AL: "Monte-Carlo dosimetry for intraoperative radiotherapy using a low energy x-ray source",ACTA ONCOLOGICA., vol. 54, no. 10, 24 March 2015 (2015-03-24), pages 1788-1795, discloses the development and validation of a Monte Carlo dose calculation platform which is configured to simulate an irradiation map based on CT-images from the intervention area of a patient. This document does not disclose or suggest the training of a CNN to predict 3D irradiation maps in real-time, as claimed. More particularly, this document does not disclose or suggest building a learning set as claimed. This document is considered to be the closest prior art.

Therefore, there is no solution in the state of the art that is both near-real time and sufficiently accurate.

### Summary of the invention

An objective of the present invention is to provide a method and a system that at least partially alleviates the above-mentioned drawbacks.

More particularly, according to embodiments, it aims to provide an irradiation map in real time, or quasi-real time, i.e. in a time allowing the practitioner to control the irradiation of the patient during the operation. In particular, the practitioner is not hindered in his operation by the time required to obtain the irradiation map. He can thus react during the operation according to the dynamically provided information.

In a first example embodiment, a method is provided for obtaining an irradiation map of a patient during interventional radiology, comprising:
a learning phase consisting in submitting to a multilayer neural network a learning set comprising associations between a first input tensor comprising data of a first radiology image of a patient's intervention area, and first acquisition parameters of an interventional radiology device, and labels corresponding to an irradiation map obtained by simulation by a simulation module from the said first radiology image and the said first acquisition parameters,
a prediction phase on a given patient, comprising the acquisition of a stream of second acquisition parameters of said interventional radiology device, the preparation of a second input tensor comprising data of a second radiology image of said given patient and of said second acquisition parameters, the submission of said second input vector to said neural network and the retrieval of an irradiation map prediction.

In preferred embodiments, the invention comprises one or more of the following features which may be used separately or in partial combination with each other or in total combination with each other:
- during the learning phase, a plurality of data of the learning set is generated for a first radiology image by varying said first acquisition parameters among possible parameters;
- said simulation is performed by a Monte-Carlo method adapted for a graphics processor:;
- said neural network is of U-Net type;
- said neural network consists of a first sub-network having as input the data of said first or second radiology image, and comprising a first succession of convolution and pooling layers and a second succession of deconvolution and convolution layers, such that the output of said neural network is of the same size as said input, and wherein the output of each deconvolution layer is concatenated with the corresponding output in said first succession; and a second subnetwork having as input said first or second acquisition parameters, respectively, and whose output is concatenated with the output of the last pooling layer of said first succession;
- said input tensor is a concatenation of said data of a first radiology image and said first acquisition parameters;
- said radiology image is a computerized tomography scan.

According to other embodiments, the invention relates to a computer readable medium encoding a machine-executable program of instructions to perform a method as previously decribed.

According to other embodiments, the invention relates to a system for obtaining a prediction for an irradiation map of a patient during interventional radiology, comprising at least one simulation module and a multilayer neural network module comprising a multilayer neural network, said system being adapted to:
in a learning phase, submitting to said multilayer neural network a learning set comprising associations between a first input tensor comprising data of a first radiology image of a patient's intervention area, and first acquisition parameters of an interventional radiology device, and labels corresponding to an irradiation map obtained from said simulation module on the basis of said first radiology image and said first acquisition parameters; and,
in a prediction phase on a given patient, acquiring a stream of second acquisition parameters from said interventional radiology device, preparing a second input tensor comprising data of a second radiology image of said given patient and said second acquisition parameters, submitting said second input vector to said neural network and retrieving a prediction of irradiation map.

Further features and advantages of the invention will become apparent from the following description of a preferred embodiment of the invention, given by way of example and with reference to the attached drawings.

### Brief Description of the Figures

Some embodiments are now described, by way of example only, and with reference to the accompagnying drawings, in which :
- figure 1 schematically represents an example of functional architecture for a learning phase according to an embodiment of the invention.
- figure 2 schematically illustrates an example of functional architecture for a prediction phase according to an embodiment of the invention
- figure 3 schematically illustrates a basic architecture of a classical neural network.
- figure 4 schematically illustrates an example of a multilayer neural network according to an embodiment of the invention.

### Description of Embodiments

One of the aims of the invention lies in obtaining an irradiation map of a patient during radiology of a given patient.

This irradiation map associates a quantity, or dose, of irradiation with points in space contained in an environment of the patient impacted by an interventional radiology. These points are more or less numerous according to a chosen sampling of the area.

The irradiation map is based on data acquired during the operation involving interventional radiology.

According to the invention, the irradiation map is obtained quickly enough to allow its production to the practitioner during the operation while remaining relevant to the data acquired. In other words, the irradiation taking place between the time of data acquisition and the production of the irradiation map can be considered negligible.

The production of the irradiation map may allow the practitioner to react to the information represented by this map during the operation. This can be referred to as real-time, in the sense that a human practitioner is able to react dynamically to the irradiation map calculated from measured data.

According to the invention, the generation of an irradiation map is based on a predictive model constituted by a multilayer neural network.

From a very high-level point of view, multi-layer neural networks can be seen as black boxes whose internal parameters must be adjusted during a learning (or training) phase by presenting them with both input data and a desired output (or "label"). The error between this desired output and the 'natural' output of the network enables the parameters to be adjusted slightly in order to reduce the error. By presenting a large number of such "input data / desired output" pairs, the network learns to react correctly and provide a good output when presented with new, unlabelled input data.

According to an embodiment of the invention, the neural network used may be a multilayer perceptron. Other neural network architectures may be possible. In particular, a convolutional neural network can be used (ConvNet or CNN).

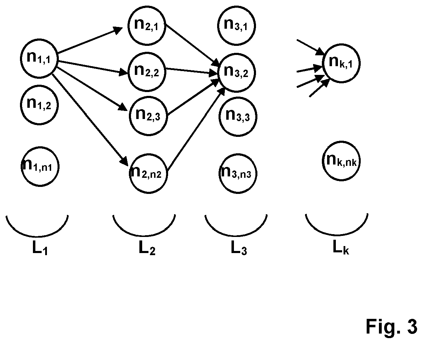

An example of a multilayer perceptron is shown in figure 3.

The multilayer perceptron (MLP) is a type of artificial neural network organised in several layers in which information flows from the input layer L₁ to the output layer Lₖ only; it is thus a feedforward network. Each layer L₁, L₂, L₃...Lₖ consists of a variable number of neurons, respectively n₁, n₂, n₃...nₖ. The neurons of the last layer (called "output") are the outputs of the neural network and represent a prediction of the model in response to an input provided on the L₁ layer.

In a multi-layer perceptron, each neuron n_{i,j} is connected as an output to all the neurons of the next layer Lᵢ₊₁. Conversely, it receives as input the outputs of all the neurons in the previous layer Lᵢ₋₁. In Figure 3, for clarity, only some connections are represented by oriented arrows.

Each connection is associated with a weight. The set of weights form the internal parameters of the neural network. They must be determined during a learning (or training) phase and then allow the prediction of output values, by generalisation, from a new input vector presented on the L₁ input layer.

Each neuron n_{i,j} classically performs a weighted sum of these inputs by the weights of the associated connections and then applies an activation function to this sum.

Several techniques exist to determine the internal parameters of the network, the thresholds, by learning. One example is the Stochastic Gradient Descent (SGD) algorithm, described for example in Le Cun, Yann A., et al. "Efficient backprop. Neural networks: Tricks of the trade", Springer Berlin Heidelberg, 2012. 9-48. Another example is ADAM, originally described in Diederik P. Kingma and Jimmy Lei Ba. "Adam: A method for stochastic optimization". 2014. arXiv:1412.6980v9, or RMSprop, described in particular in Tijmen Tieleman and Geoffrey Hinton, "Lecture 6.5-rmsprop: Divide the gradient by a running average of its recent magnitude", COURSERA: neural networks for machine learning, 4(2):26-31, 2012.

Also, the process of obtaining an irradiation map includes a learning phase allowing to determine the internal parameters of the multilayer neural network.

Once these have been determined, the multilayer neural network constitutes a predictive model that can be used in a prediction phase during an intervention (or operation).

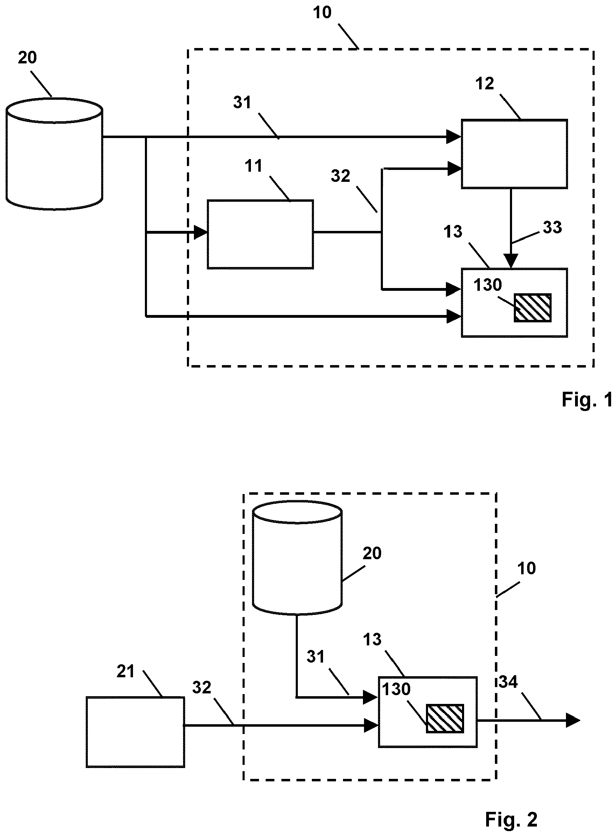

Figure 1 illustrates such a learning phase according to an embodiment of the invention.

According to the invention, the learning phase consists of submitting the multilayer neural network 13 to a learning set comprising associations between a learning input tensor itself comprising data from a radiology image 31 of a patient's intervention area, and acquisition parameters 32 of the interventional radiology device, and labels corresponding to an irradiation map 33 obtained by simulation, by a simulation module 12, on the basis of the radiology image 31 and of these acquisition parameters 32. The learning set may stored in a database or data repository 20.

In a conventional manner, this learning phase is carried out before and outside the operation involving interventional radiology.

The learning phase is based on a set of radiology images.

According to embodiments, the radiology images are three-dimensional images.

However, according to some other embodiments, other options may be contemplated. For instance, 2D images may be considered, the volume of the patient's intervention area captured by the radiology device being represented by a set of 2D images, each of them representing a layer of the volume.

Also, 4D images may be considered, the forth dimension being the time (video stream).

Experiments have shown that it is fasted to predict a 3D image once by a multilayer neural network than to predict sequentially a serie of 2D images composing a 3D image.

In the following, the wording "three dimensional images" will be preferred, but the scope of the patent encompasses 2D and 4D images as well.

The radiology images (e.g. three-dimensional images) captures an intervention area of the patient related to the interventional radiology, i.e. locations of the patient's body where the interventional radiology is aimed to produce effects.

These radiology images (e.g. three-dimensional images) may be of different kinds. For example, it may be a CT scan or computerised tomography (CT). This is a medical imaging technique which consists of measuring the absorption of X-rays by tissues and then, by computer processing, digitising and finally reconstructing 2D or 3D images of anatomical structures. To acquire the data, the tomographic or "slice" analysis technique is used, by subjecting the patient to the scanning of an X-ray beam.

Typically, such a 3D image is taken prior to any intervention (or operation) in order to plan it. It is therefore possible to easily acquire a large number of such 3D images to form the training set.

Moreover, for each of these 3D images, acquisition parameters 32 of the interventional radiology device to be used for the operation can be associated. The interventional radiology device is designed to acquire an image, generally two-dimensional, of a patient's intervention area, that is a function of these acquisition parameters.

In practice, the interventional radiology device generates a two-dimensional image stream for the practitioner. It should be noted that these images are distinct from the one, 31, acquired before the intervention.

The acquisition parameters are primarily the three-axis coordinates, x,y,z, of the interventional radiology device, the angles α, β of orientation of the device, and the voltage, KV, of the device's tube. α may represent rotation about the longitudinal axis of the patient while β may represent rotation about the horizontal axis.

Depending on the type of interventional radiology device, other parameters may be used.

According to the invention, an input tensor of the neural network 13 may be formed by concatenating data from a CT scan image and acquisition parameters (x, y, z, α, β, KV), 32.

According to an embodiment of the invention, for each three-dimensional image 31, a plurality of training set data is generated by varying the acquisition parameters (x, y, z, α, β, KV) among possible parameters.

To do this, excursion intervals can be defined for each of the parameters x, y, z, α, β, KV as well as respective sampling rates. Thus, a large number of possible parameterizations can be defined for the same image for which an irradiation map 33 can be simulated.

Each three-dimensional image 31/acquisition parameters 32 pair can be produced at the input of a simulator 12 which provides a simulated irradiation map 33 at the output. This irradiation map 33 may be submitted as a label to the neural network 13.

In other words, at each iteration of the learning phase, the neural network 12, is provided with:
- an input tensor comprising a three-dimensional image 31 and acquisition parameters 32 ;
- associated with a label consisting of an irradiation map 33 simulated on the basis of this same three-dimensional image 31 and these same acquisition parameters 32.

In other words, the neural network allows correlation between the three-dimensional image data and the acquisition parameters (x, y, z, α, β, KV), 32 with corresponding acquisition maps.

According to the invention, the input tensor of the neural network contains only data produced by the interventional radiology device. No complex pre-processing needs to be performed in order to transform this acquired data into other data (apart from possible simple pre-processing such as re-scaling, re-sampling, etc. which do not change the nature of the data).

As a result, the training set is inexpensive to build and a large number of labelled examples can be produced by simply retrieving three dimensional images. This training set can be further enriched by varying the acquisition parameters according to an embodiment of the invention.

Unlike the method described in the Roser et al. paper mentioned above, it is neither necessary to deploy costly pre-processing that would slow down the constitution of a sufficiently large learning base, nor to obtain voxelized 3D models of each patient organ. The latter requirement is indeed detrimental to the constitution of a large learning base.

However, it is important that the training base is large enough to allow the neural network to converge to a stable state that minimises its cost function, so that it can train an effective predictive model. A training base that is too small will either give erroneous or very inaccurate predictions, or correct predictions but on examples that differ only slightly from those in the training set (reduced generalisation capacity).

Thus, even with the invention, it is possible to take advantage of available databases collecting patient data in order to perform this learning phase.

For example, a database of CT scan images of the pancreas exists and is documented in the article H. R. Roth, L. Lu, A. Farag, H.-C. Shin, J. Liu, E. Turkbey, R. M. Summers, "Data from Pancreas-CT", 2016. This database contains 82 images of 512 x 512 pixels, spaced in depth between 0.7 and 1 mm. From this database, three-dimensional images with a voxel resolution of 4 min x 4 min x 2 min can be redefined.

A simulation module 12 may perform a simulation of the behaviour of the particles emitted by the interventional radiology device according to its acquisition parameters 32 in the patient's intervention area as defined by the three dimensional image 31.

This simulation may be a simulation according to a Monte-Carlo method.

In particular, it may be a simulation according to a Monte Carlo method based on a GPU. For example, the GGEMS platform, for "GPU GEant4-based Monte Carlo Simulation", as described in the article by J. Bert, H. Perez-Ponce, Z. El Bitar, S. Jan, Y. Boursier, et al, "Geant4-based Monte Carlo simulations on GPU for medical applications" in Physics in Medicine and Biology, IOP Publishing, 2013, 58, pp.5593-5611, can be used. The objective of GGEMS is to provide a flexible platform for the simulation of various medical applications. The simulation algorithm can also be modified to better fit a platform running on a GPU.

In a concrete case of experimentation of the method of the invention, the source constituted by the interventional radiology device can be defined as forming a conical radiation of KV value. For each simulation, the behaviour of 10⁷ particles can be simulated in a time of about 6 seconds on a GPU of the NVDIA 1080 GTX Ti type.

According to an embodiment of the invention, the acquisition parameters can be varied for each image in order to increase the population of the resulting training set.

Typically, the angles α, β vary in the ranges [-50°, 90°] and [-30°, +30°] respectively with steps of 2°.

The position of the interventional radiology device can vary in the intervals [-30cm; +30cm] for the three parameters x, y, z, with steps of 5 cm.

Finally, the tube voltage KV can be varied in the range [80kV, 120kV] with a step of 10kV.

These values of intervals and steps have been determined by studying the technical data of interventional radiology devices and by feedback from practitioners (surgeons, radiologists, etc.).

The simulation module 12 provides an irradiation map 33. This radiation map is a three-dimensional image that associates points in space within a simulated environment with radiation doses.

The simulated radiation maps 33 form labels for the neural network 130 implemented by a neural network module 13. In a manner known per se, training involves calculating an output of the neural network 130 as a function of the input tensor to measure a cost function that estimates a distance between that output and the associated label, or irradiation map, that is desired output. The difference between these two values (in this case two tensors), evaluated by the cost function, is used to modify the parameters of the neural network in general by a gradient descent method as previously explained.

According to an embodiment of the invention, the cost function may be the evaluation of the mean square error between the output tensor of the neural network 13 and the desired output represented by the irradiation map (labels).

The learning mechanism can be optimized for example by the ADAM algorithm, described in D. P. Kingma and J. Ba, "Adam: A Method for Stochastic Optimization", CoRR, abs/1412.6980 (2014).

The neural network 13 may be implemented on a TensorFlow platform using the Keras API.

According to an embodiment of the invention, the neural network is a convolutional self-encoding multilayer neural network.

More particularly, the neural network may be of the u-net type.

The u-net neural network is a convolutional network, first described in Olaf Ronneberger, Philipp Fischer, Thomas Brox, "U-net: Convolutional networks for biomedical image segmentation", in International Conference on Medical Image Computing and Computer-Assisted Intervention, pages 234-241, Springer, 2015.

A 3D version was then proposed in O. Cicek, A. Abdulkadir, S. S. Lienkamp, T. Brox, and O. Ronneberger, "3D U-net: learning dense volumetric segmentation from sparse annotation" in Lect. Ronneberger, "3D U-net: learning dense volumetric segmentation from sparse annotation" in Lect. Notes Computing Science, vol. 9901 LNCS, pages 424-432, 2016.

According to an embodiment of the invention, the neural network is based on the 3D U-net architecture.

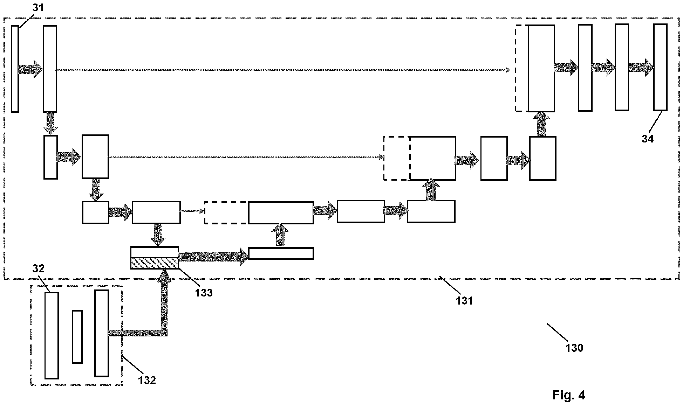

Figure 4 illustrates an example of such an embodiment.

In particular, the neural network 130 of the neural network module 13 is composed of a first subnet 131 corresponding to a 3D u-net and a second subnet 132 whose output is concatenated within the first subnet.

The first sub-network 131 itself comprises a first succession of layers constituting an encoding path (or contraction, or subsampling) and a second succession of layers constituting a decoding path (or expansion, or oversampling).

The encoding path is a succession of convolution layers and pooling layers.

More specifically, the encoding path may comprise successions of two successive 3x3x3 convolution layers, each followed by a ReLU activation and a 2x2x2 maxPooling layer. At each subsampling (or encoding) stage, the number of filters (or features) is doubled, starting with 8 for the first convolution.

The first layer of this encoding path takes as input the tensor formed by the three-dimensional image data 31.

The pooling operation reduces the dimensionality and captures the most relevant filters or features.

In Figure 4, the horizontal arrows to the right correspond to these convolution operations and the downward arrows correspond to maxPooling operations.

Similarly, the decoding path (right in Figure 4) has a succession of deconvolution and convolution layers.

The deconvolutions (or transposed convolutions, or oversampling), for example of 2x2x2 are represented by the upward arrows, while the convolutions, for example of 3x3x3 are represented by the rightward arrows in the same way as for the decoding channel.

In this way, an output (i.e. the irradiation map 34) of the same size as the input 31 can be obtained.

This neural network is related to the family of encoder-decoder networks, but unlike conventional architectures, there is a coupling between the encoding and decoding paths. More precisely, the output of each deconvolution layer is concatenated with the corresponding output in said first succession. This coupling is represented by the thin arrows in Figure 4 and the empty blocks representing the output of the deconvolution layers to be concatenated into the decoding path.

This coupling allows the transfer of fine-grained information from the low-level layers of the encoding path to the high-level layers of the decoding path, as this information allows the generation of reconstructions with fine detail.

As illustrated in the figure 4, the pluralities of pooling (forming a subsampling) and then oversampling operations give the network a U-shaped (functional) form which gives it its usual name of "U-net".

The last layers of the encoding path and the first layers of the decoding path can also be considered as forming a bottleneck.

According to this embodiment, the neural network comprises, in an original way, a second subnetwork 132 which is coupled with the first subnetwork 131 at the level of this bottleneck.

This second subnet 132 takes as input the acquisition parameters 32 of the interventional radiology device.

This second sub-network may architecturally be a network of a few fully connected layers (here 3 in Figure 4). The output tensor 133 is concatenated with the output of the last pooling layer of the encoding path. This tensor has the same number of units (19x12x15=3420) as the output tensor of this last pooling layer in order to allow their concatenation.

According to an embodiment, the number of filters (or "features") for the convolution and deconvolution operations have been empirically reduced by a factor of 8 compared to the original 3D u-net, in order to allow faster predictions (in the prediction phase) compatible with a real-time requirement. The idea of doubling the number of filters before pooling operations has however been retained.

According to one embodiment, another distinctive feature compared to the u-net 3D scheme is the use of a padding mechanism to obtain an output of the same size as the input.

Padding aims in avoiding the edge effect of the recurrent convolution processes applied to the images.

More precisely, pixels around the image edge can not be process due to the convolution.

For example, in case of a 3x3x3 convolutional filter, the edge around the image after processing is cropped with a size of 1 pixel. Consequently, the size of the image is reduced by two pixels on each dimension after every convolution, and it is not possible to recover this dimension on the decoder path.

This may be avoided by applying zero-padding to the input image before each convolution operation. The zero-padding consists of adding pixels with zeros value around the edge of the image.

In order to optimise the learning time, different mechanisms can be implemented.

For example, the training can be divided into two sub-phases.

In a first sub-phase, only a subset of the plurality of training set data obtained by varying the acquisition parameters among possible parameters is used. This subset is substantially smaller than this plurality.

For example, the module 11 generates in this sub-phase only a smaller number of parameters but preferably evenly distributed, for example using a larger step size.

For example, the orientation angles vary in 10° steps; the x,y,z position of the source varies in 15 cm steps and the tube voltage KV varies in 40kV steps.

This first sub-phase allows the state of the neural network to quickly converge to an acceptable state.

In a second sub-phase, the entire training set is used (i.e. with smaller steps, as previously indicated).

The training set is specific to a particular area of intervention: for example, specific training sets may be formed for the head and neck, for the thorax, for the pelvis, etc. A training set may be formed for the head and neck, for the thorax, for the pelvis, etc. A training set can also be formed for the whole body.

Each training set leads to a specific state of the neural network 13. This state is materialized by the values of all its parameters (synaptic weights, etc.) and thus constitutes a specific predictive model.

Once constituted, these predictive models (state of the neural network 13) can be stored for use in the prediction phase.

During a prediction phase, a first step may therefore be to choose the appropriate predictive model from a library of predictive models. This step can be manual: the practitioner chooses the intervention area, which leads to the selection of the associated predictive model. According to other embodiments, this step may be automatic by applying image recognition algorithms allowing automatic recognition of an area of intervention.

This prediction phase consists in applying the predictive model for a given patient in order to predict a real-time radiation map to help or guide the practitioner during interventional radiology.

This phase is illustrated in Figure 2.

During the intervention, the interventional radiology device 21 generates an acquisition parameter stream 32. This stream is determined, for example, by a sampling rate at which it transmits the acquisition parameters to the radiation map generation system, 10 radiology.

The parameters may be the 6-tuple previously described (x,y,z,α,β,KV), or any set of parameters specific to the type of radiology device. In any case, these parameters must be the same as those used for the learning phase.

These parameters evolve over time during the intervention, depending on the actions of the practician. Their values influence the irradiation of the patient, in particular the area of the patient for the parameters (x,y,z,α,β) and the instantaneous dose for the parameter KV.

Furthermore, the system 10 is provided to prepare an input tensor comprising data of a radiology image 31 of the given patient, e.g. a three-dimensional. This radiology image is of the same nature as those used in the learning phase. It may be a pre-operative image of the patient, stored in the data repository 20, or an image acquired during the interventional radiology operation. According to the invention, this image can be unique during the operation (only the parameters change over time).

The input tensor also includes parameters for acquiring the flow generated by the interventional radiology device 21.

Sampling may be applied to take only a sampled portion of the flow to construct this input tensor. The sampling may be determined based on the computation time for each prediction.

Once this input tensor has been prepared and submitted as an input to the neural network 13, a prediction of the irradiation map 34 can be retrieved as an output from the neural network 13.

This irradiation map 34 is of the same type as those provided as labels during the learning phase. In particular, it may provide an estimated radiation dose per unit volume of the patient's intervention area during the interventional radiology operation.

This irradiation map can be seen as a 3D image in which each voxel is associated with an irradiation dose (instead of a colorimetric data as in a classical 3D image).

Experimental measurements carried out by the inventors have made it possible to estimate that an irradiation map takes less than 70 ms, with a standard GPU graphics card.

This irradiation map can be used to assist the practitioner during his intervention on the patient.

In particular, it can be displayed on a screen so that the practician can view it during the procedure.

Radiation doses can be represented by associating different colours for intervals of dose values.

Since the time to calculate a new map is extremely short, the display can be refreshed (about 15 maps per second), so that the map displayed corresponds to the present time for the practician, allowing him/her to monitor the map in real time.

Also, alerts (visual, acoustic...) can be triggered if radiation doses exceed certain predetermined thresholds for certain sub-areas of the intervention area.

For example, it may be possible to visually highlight areas that may be problematic, either because the associated radiation dose has exceeded a threshold or because it shows too much radiation dose growth, suggesting that the threshold may be exceeded in the future.

Contrary to some prior art proposals, the predicted radiation map is a 3D map. In other words, an estimate of the radiation dose can be obtained not only for the skin surface but also for the whole volume of the irradiated organs and body parts.

Furthermore, the error inherent in a neural network prediction is very reasonable and less than 5% compared to a reference Monte Carlo simulation.

Moreover, as mentioned previously, the method uses only medical imaging data that is easy to obtain in the training phase, so that training sets can be constituted for various parts of the body that are sufficiently large to allow the neural network to converge well and thus obtain good qualitative performance in the prediction phase.

In one embodiment, the system 10 may be implemented by a set of circuits co-located in a centralized server or distributed within a distributed server or among a set of servers. This set of servers may comprise "server farm" or "cloud computing" arrangements. This server may also be implemented on a single computer located in the operating room.

This system 10 may have means of communication with remote servers in order to retrieve three-dimensional images of patients, both in the learning phase and in the prediction phase (in order to obtain a pre-operative image of the patient on whom the operation is to be performed).

The system may also have means of communication with the interventional radiology device 21 in order, in particular, to retrieve the acquisition parameters.

These communication means may be in accordance with the technologies of the state of the art. In particular, they may be wired (Ethernet, USB, etc.) or radio (Wi-Fi, Bluetooth, 3G/4G/5G cellular, etc.) communication means.

## Claims

1. Computer implemented method for training a multilayer neural network (130) in view of obtaining an irradiation map of an intervention area of a patient during interventional radiology using an interventional radiology device (21), said method comprising:
- a learning phase consisting in submitting to the multilayer neural network (130) a learning set comprising first input tensors associated with labels, each of said first input tensors comprising data of a first 3D radiology image (31) of a patient in the intervention area and data of first acquisition parameters (32) of the interventional radiology device, said first acquisition parameters comprising three-axis coordinates (x,y,z) of the interventional radiology device, angles, α, β, of orientation of the device, and voltage (KV) of a tube of the interventional radiology device, α representing rotation about a longitudinal axis of the patient, β a rotation about an horizontal axis, each of said labels consisting of an irradiation map (33) obtained by simulation by a simulation module (12) from the said first 3D radiology image (31) and the said first acquisition parameters (32), said irradiation map comprising a 3D image of the intervention area, said 3D image comprising voxels associated with a simulated irradiation dose per unit volume of the intervention area, a predictive model specific to the intervention area being constituted,
- storing the predictive model, for use by said trained multilayer neural network for obtaining as output an irradiation map prediction (34) of said given patient in said intervention area during interventional radiology, in response to submitting to said neural network a second input tensor, said second input tensor comprising data of a second 3D radiology image (31) of said patient in the intervention area, and data of second acquisition parameters acquired during the interventional radiology from the interventional radiology device (21), said second 3D radiology image and said second acquisition parameters being of a same type as those used in the learning phase, said irradiation map prediction being of a same type as the labels of the learning set.

2. A method according to the preceding claim, wherein, during the learning phase, a plurality of data of the learning set is generated (11) for a first radiology image (31) by varying said first acquisition parameters among possible parameters.

3. The method according to any of the preceding claims, wherein said simulation is performed by a Monte-Carlo method adapted for a graphics processor.

4. The method according to any of the preceding claims, wherein said neural network is of U-Net type.

5. A method according to the preceding claim, wherein said neural network (130) consists of a first sub-network (131) having as input the data of said first or second radiology image, and comprising a first succession of convolution and pooling layers and a second succession of deconvolution and convolution layers, such that the output of said neural network (130) is of the same size as said input, and wherein the output of each deconvolution layer is concatenated with the corresponding output in said first succession; and a second sub-network (132) having as input said first or second acquisition parameters, respectively, and whose output is concatenated with the output of the last pooling layer of said first succession.

6. A method according to any of the preceding claims, wherein said input tensor is a concatenation of said data of a first radiology image (31) and said first acquisition parameters (32).

7. A method according to any of the preceding claims, wherein said radiology image (31) is a computerized tomography scan.

8. A computer readable medium encoding a machine-executable program of instructions to perform a method according to any one of claims 1 to 7.

9. A system (10) for obtaining a prediction for an irradiation map (34) of a patient in an intervention area during interventional radiology, comprising at least one simulation module (12) and a multilayer neural network module (13) comprising a multilayer neural network (130), said system being adapted to
- obtain a stream of acquisition parameters (32) from an interventional radiology device (21), said acquisition parameters comprising three-axis coordinates (x,y,z) of the interventional radiology device, angles, α, β, of orientation of the device, and voltage (KV) of a tube of the interventional radiology device, α representing rotation about a longitudinal axis of the patient, β a rotation about an horizontal axis,
- obtain a 3D radiology image (31) of the patient (130) in the intervention area,
- prepare an input tensor comprising data of the 3D radiology image and of the acquisition parameters,
- submit said input tensor as an input to the multilayer neural network (13), said neural network having been trained during a learning phase with a learning set of first input tensors associated with labels, each of said first input tensors being of a same type as said input tensor prepared for the patient and comprising data of a first 3D radiology image (31) of a patient in the intervention area, and first acquisition parameters (32) of the interventional radiology device, each of said labels corresponding to an irradiation map (33) obtained from said simulation module (12) on the basis of said first radiology image (31) and said first acquisition parameters (32); and comprising a 3D image of the intervention area comprising voxels associated with a simulated irradiation dose per unit volume of the intervention area,
- retrieve as an output of the multilayer neural network (13) an irradiation map of the patient in the intervention area, said irradiation map being of a same type as the labels of the learning set.

## Patentansprüche

1. Computerimplementiertes Verfahren zum Trainieren eines mehrschichtigen neuronalen Netzwerks (130) im Hinblick auf ein Erhalten einer Bestrahlungskarte eines Interventionsbereichs eines Patienten während einer interventionellen Radiologie unter Verwendung einer Vorrichtung (21) für interventionelle Radiologie, das Verfahren umfassend:
- eine Lernphase, die darin besteht, dass dem mehrschichtigen neuronalen Netzwerk (130) ein Lernsatz vorgelegt wird, umfassend erste Eingabetensoren, die Etiketten zugeordnet sind, jeder der ersten Eingabetensoren umfassend Daten eines ersten 3D-Radiologie-Bilds (31) eines Patienten in dem Interventionsbereich und Daten erster Erfassungsparameter (32) der Vorrichtung für interventionelle Radiologie, die ersten Erfassungsparameter umfassend dreiachsige Koordinaten (x,y,z) der Vorrichtung für interventionelle Radiologie, Winkel, α, β, einer Ausrichtung der Vorrichtung und Spannung (KV) eines Schlauchs der Vorrichtung für interventionelle Radiologie, wobei α eine Drehung um eine Längsachse des Patienten herum, β eine Drehung um eine horizontale Achse herum darstellt, jedes der Etiketten bestehend aus einer Bestrahlungskarte (33), die durch eine Simulation durch ein Simulationsmodul (12) aus dem ersten 3D-Radiologie-Bild (31) und den ersten Erfassungsparametern (32) erhalten wird, die Bestrahlungskarte umfassend ein 3D-Bild des Interventionsbereichs, das 3D-Bild umfassend Voxel, die einer simulierten Bestrahlungsdosis pro Volumeneinheit des Interventionsbereichs zugeordnet sind, wobei ein Vorhersagemodell, das für den Interventionsbereich spezifisch ist, gebildet wird,
- Speichern des Vorhersagemodells zur Verwendung durch das trainierte mehrschichtige neuronale Netzwerk zum Erhalten als eine Ausgabe einer Bestrahlungskartenvorhersage (34) des gegebenen Patienten in dem Interventionsbereich während der interventionellen Radiologie, als Reaktion auf das Vorlegen eines zweiten Eingabetensors an das neuronale Netzwerk, der zweite Eingabetensor umfassend Daten eines zweiten 3D-Radiologie-Bilds (31) des Patienten in dem Interventionsbereich und Daten von zweiten Erfassungsparametern, die während der interventionellen Radiologie aus der-Vorrichtung (21) für interventionelle Radiologie erfasst werden, wobei das 3D-Radiologie-Bild und die zweiten Erfassungsparameter von einer gleichen Art wie diejenigen sind, die in der Lernphase verwendet werden, wobei die Bestrahlungskartenvorhersage von einer gleichen Art wie die Etiketten des Lernsatzes ist.

2. Verfahren nach dem vorstehenden Anspruch, wobei, während der Lernphase, eine Vielzahl von Daten des Lernsatzes für ein erstes Radiologiebild (31) durch Variieren der ersten Erfassungsparameter unter möglichen Parametern erzeugt (11) wird.

3. Verfahren nach einem der vorstehenden Ansprüche, wobei die Simulation durch ein Monte-Carlo-Verfahren durchgeführt wird, das für einen Grafikprozessor angepasst ist.

4. Verfahren nach einem der vorstehenden Ansprüche, wobei das neuronale Netzwerk von einer U-Net-Art ist.

5. Verfahren nach dem vorstehenden Anspruch, wobei das neuronale Netzwerk (130) aus einem ersten Teilnetzwerk (131) besteht, das Daten des ersten oder des zweiten Radiologiebilds als die Eingabe aufweist, und umfassend eine erste Abfolge von Faltungs- und Pooling-Schichten und eine zweite Abfolge von Entfaltungs- und Faltungsschichten derart, dass die Ausgabe des neuronalen Netzwerks (130) von der gleichen Größe wie die Eingabe ist, und wobei die Ausgabe jeder Entfaltungsschicht mit der entsprechenden Ausgabe in der ersten Abfolge verkettet ist; und ein zweites Teilnetzwerk (132), das die ersten beziehungsweise die zweiten Erfassungsparameter als die Eingabe aufweist und dessen Ausgabe mit der Ausgabe der letzten Pooling-Schicht der ersten Abfolge verkettet wird.

6. Verfahren nach einem der vorstehenden Ansprüche, wobei der Eingabetensor eine Verkettung der Daten eines ersten Radiologiebilds ist (31) mit den ersten Erfassungsparametern (32) ist.

7. Verfahren nach einem der vorstehenden Ansprüche, wobei das Radiologiebild (31) ein computergestützter Tomografiescan ist.

8. Computerlesbares Medium, das ein maschinenausführbares Programm von Anweisungen codiert, um ein Verfahren nach einem der Ansprüche 1 bis 7 durchzuführen.

9. System (10) zum Erhalten einer Vorhersage für eine Bestrahlungskarte (34) eines Patienten in einem Interventionsbereich während der interventionellen Radiologie, umfassend mindestens ein Simulationsmodul (12) und ein mehrschichtiges neuronales Netzwerkmodul (13) umfassend ein mehrschichtiges neuronales Netzwerk (130), wobei das System angepasst ist zum
- Erhalten eines Stroms von Erfassungsparametern (32) aus einer Vorrichtung (21) für interventionelle Radiologie, die Erfassungsparameter umfassend dreiachsige Koordinaten (x,y,z) der Vorrichtung für interventionelle Radiologie, Winkel, α, β, der Ausrichtung der Vorrichtung und Spannung (KV) eines Schlauchs der Vorrichtung für interventionelle Radiologie, wobei α die Drehung um eine Längsachse des Patienten herum, β eine Drehung um eine horizontale Achse herum darstellt,
- Erhalten eines 3D-Radiologie-Bilds (31) des Patienten (130) in dem Interventionsbereich,
- Vorbereiten eines Eingabetensors, umfassend Daten des 3D-Radiologie-Bilds und der Erfassungsparameter,
- Vorlegen des Eingabetensors als eine Eingabe in das mehrschichtige neuronale Netzwerk (13), wobei das neuronale Netzwerk während einer Lernphase mit einem Lernsatz von ersten Eingabetensoren trainiert wurde, die Etiketten zugeordnet sind, wobei jeder der ersten Eingabetensoren von einer gleichen Art wie der für den Patienten vorbereitete Eingabetensor ist, und umfassend Daten eines ersten 3D-Radiologie-Bilds (31) eines Patienten in dem Interventionsbereich und erste Erfassungsparameter (32) der Vorrichtung für interventionelle Radiologie, wobei jedes der Etiketten einer Bestrahlungskarte (33) entspricht, die aus dem Simulationsmodul (12) auf der Basis des ersten Radiologiebilds (31) und der ersten Erfassungsparameter (32) erhalten wird; und umfassend ein 3D-Bild des Interventionsbereichs, umfassend Voxel, die einer simulierten Bestrahlungsdosis pro Volumeneinheit des Interventionsbereichs zugeordnet sind,
- Abrufen als eine Ausgabe des mehrschichtigen neuronalen Netzwerks (13) einer Bestrahlungskarte des Patienten in dem Interventionsbereich, wobei die Bestrahlungskarte von einer gleichen Art wie die Etiketten des Lernsatzes ist.

## Revendications

1. Procédé mis en oeuvre par ordinateur pour entraîner un réseau neuronal multicouche (130) en vue d'obtenir une carte d'irradiation d'une zone d'intervention d'un patient pendant la radiologie interventionnelle à l'aide d'un dispositif de radiologie interventionnelle (21), ledit procédé comprenant :
- une phase d'apprentissage consistant à soumettre au réseau neuronal multicouche (130) un ensemble d'apprentissage comprenant des premiers tenseurs d'entrée associés à des étiquettes, chacun desdits premiers tenseurs d'entrée comprenant des données d'une première image radiologique 3D (31) d'un patient dans la zone d'intervention et des données de premiers paramètres d'acquisition (32) du dispositif de radiologie interventionnelle, lesdits premiers paramètres d'acquisition comprenant des coordonnées triaxiales (x, y,z) du dispositif de radiologie interventionnelle, des angles, α, β, d'orientation du dispositif, et une tension (KV) d'un tube du dispositif de radiologie interventionnelle, α représentant une rotation autour d'un axe longitudinal du patient, β une rotation autour d'un axe horizontal, chacune desdites étiquettes étant constituée d'une carte d'irradiation (33) obtenue par simulation par un module de simulation (12) à partir de ladite première image radiologique 3D (31) et desdits premiers paramètres d'acquisition (32), ladite carte d'irradiation comprenant une image 3D de la zone d'intervention, ladite image 3D comprenant des voxels associés à une dose d'irradiation simulée par unité de volume de la zone d'intervention, un modèle prédictif propre à la zone d'intervention étant constitué,
- le stockage du modèle prédictif, pour une utilisation par ledit réseau neuronal multicouche entraîné pour obtenir en sortie une prédiction de carte d'irradiation (34) dudit patient donné dans ladite zone d'intervention pendant la radiologie interventionnelle, en réponse à la soumission audit réseau neuronal d'un second tenseur d'entrée, ledit second tenseur d'entrée comprenant des données d'une seconde image radiologique 3D (31) dudit patient dans la zone d'intervention, et des données de seconds paramètres d'acquisition acquises pendant la radiologie interventionnelle à partir du dispositif de radiologie interventionnelle (21), ladite seconde image radiologique 3D et lesdits seconds paramètres d'acquisition étant de même type que ceux utilisés dans la phase d'apprentissage, ladite prédiction de carte d'irradiation étant du même type que les étiquettes de l'ensemble d'apprentissage.

2. Procédé selon la revendication précédente, dans lequel, pendant la phase d'apprentissage, une pluralité de données de l'ensemble d'apprentissage est générée (11) pour une première image radiologique (31) en faisant varier lesdits premiers paramètres d'acquisition parmi des paramètres possibles.

3. Procédé selon l'une quelconque des revendications précédentes, dans lequel ladite simulation est effectuée par une méthode de Monte-Carlo adaptée à un processeur graphique.

4. Procédé selon l'une quelconque des revendications précédentes, dans lequel ledit réseau neuronal est de type U-Net.

5. Procédé selon la revendication précédente, dans lequel ledit réseau neuronal (130) est constitué d'un premier sous-réseau (131) ayant en entrée les données de ladite première ou seconde image radiologique, et comprenant une première succession de couches de convolution et de test groupé et une seconde succession de couches de déconvolution et de convolution, de sorte que la sortie dudit réseau neuronal (130) présente la même taille que ladite entrée, et dans lequel la sortie de chaque couche de déconvolution est concaténée avec la sortie correspondante dans ladite première succession ; et un second sous-réseau (132) ayant comme entrée lesdits premiers ou seconds paramètres d'acquisition, respectivement, et dont la sortie est concaténée avec la sortie de la dernière couche de test groupé de ladite première succession.

6. Procédé selon l'une quelconque des revendications précédentes, dans lequel ledit tenseur d'entrée est une concaténation desdites données d'une première image radiologique (31) et desdits premiers paramètres d'acquisition (32).

7. Procédé selon l'une quelconque des revendications précédentes, dans lequel ladite image radiologique (31) est un examen de tomographie informatisée.

8. Support lisible par ordinateur encodant un programme exécutable par machine d'instructions pour mettre en oeuvre un procédé selon l'une quelconque des revendications 1 à 7.

9. Système (10) permettant d'obtenir une prédiction pour une carte d'irradiation (34) d'un patient dans une zone d'intervention pendant la radiologie interventionnelle, comprenant au moins un module de simulation (12) et un module de réseau neuronal multicouche (13) comprenant un réseau neuronal multicouche (130), ledit système étant adapté pour
- obtenir un flux de paramètres d'acquisition (32) à partir d'un dispositif de radiologie interventionnelle (21), lesdits paramètres d'acquisition comprenant des coordonnées triaxiales (x, y,z) du dispositif de radiologie interventionnelle, des angles α, β, d'orientation du dispositif, et une tension (KV) d'un tube du dispositif de radiologie interventionnelle, α représentant une rotation autour d'un axe longitudinal du patient, β une rotation autour d'un axe horizontal,
- obtenir une image radiologique 3D (31) du patient (130) dans la zone d'intervention,
- préparer un tenseur d'entrée comprenant des données de l'image radiologique 3D et des paramètres d'acquisition,
- soumettre ledit tenseur d'entrée en entrée du réseau neuronal multicouche (13), ledit réseau neuronal ayant été entraîné lors d'une phase d'apprentissage avec un ensemble d'apprentissage de premiers tenseurs d'entrée associés à des étiquettes, chacun desdits premiers tenseurs d'entrée étant du même type que ledit tenseur d'entrée préparé pour le patient et comprenant des données d'une première image radiologique 3D (31) d'un patient dans la zone d'intervention, et des premiers paramètres d'acquisition (32) du dispositif de radiologie interventionnelle, chacune desdites étiquettes correspondant à une carte d'irradiation (33) obtenue à partir dudit module de simulation (12) en fonction de ladite première image radiologique (31) et desdits premiers paramètres d'acquisition (32) ; et comprenant une image 3D de la zone d'intervention comprenant des voxels associés à une dose d'irradiation simulée par unité de volume de la zone d'intervention,
- récupérer en sortie du réseau neuronal multicouche (13) une carte d'irradiation du patient dans la zone d'intervention, ladite carte d'irradiation étant du même type que les étiquettes de l'ensemble d'apprentissage.
